# EUROPEAN PATENT SPECIFICATION

(11) **EP 2 616 454 B1**
(45) Date of publication and mention of the grant of the patent: **15.04.2015**
(21) Application number: 11754425.4
(22) Date of filing: 12.09.2011
(51) Int. Cl.: C07D 319/06, C07D 319/08

(54) **ESTERS OF HEXANOIC ACIDS AS INTERMEDIATES FOR THE PREPARATION OF ATORVASTATIN**
HEXANSÄUREESTER ALS ZWISCHENPRODUKTE ZUR HERSTELLUNG VON ATORVASTATIN
ESTERS D'ACIDES HEXANOÏQUES COMME INTERMÉDIAIRES POUR LA PRÉPARATION D'ATORVASTATINE

(30) Priority: 16.09.2010 EP 10177138
(43) Date of publication of application: 24.07.2013
(73) Proprietor: DSM Sinochem Pharmaceuticals Netherlands B.V., 2613 AX Delft (NL)
(72) Inventor: LANGE, DE, Ben, NL-6151 GE Munstergeleen (NL); ELSENBERG, Henricus Leonardus Marie, NL-5935 CD Steyl (NL); VAESSEN, Henricus Wilhelmus Leonardus Marie, NL-6363 AL Wijnandsrade (NL)
(74) Representative: De Vroom, Erik
(86) International application number: PCT/EP2011/065706
(87) International publication number: WO 2012/034958

(56) References cited:
- WO-A1-2004/096788
- WO-A1-2008/075165
- WO-A1-2009/093776
- WO-A2-89/07598
- DATABASE CA [Online] CHEMICAL ABSTRACTS SERVICE, COLUMBUS, OHIO, US; 17 July 2004 (2004-07-17), REDDY, MANNE SATYANARAYANA ET AL: "A process for the production of amorphous atorvastatin calcium", XP002614668, retrieved from STN Database accession no. 145:505324 & IN 193 427 A1 (DR. REDDY'S LABORATORIES LIMITED, INDIA) 17 July 2004 (2004-07-17)

## Description

### Field of the invention

The present invention relates to novel esters of hexanoic acids as intermediates for the production of atorvastatin.

### Background of the invention

Atorvastatin ([*R*-(*R**,*R**)]-2-(4-fluorophenyl)-β,δ-dihydroxy-5-(1-methylethyl)-3-phenyl-4-[(phenylamino)carbonyl]-1*H*-pyrrole-1-heptanoic acid hemi calcium salt, (2)) is a pharmaceutical ingredient useful as an inhibitor of the enzyme 3-hydroxy-3-methylglutaryl-coenzyme A reductase (HMG-CoA reductase) and thus useful as a hypolipidemic and hypocholesterolemic agent.

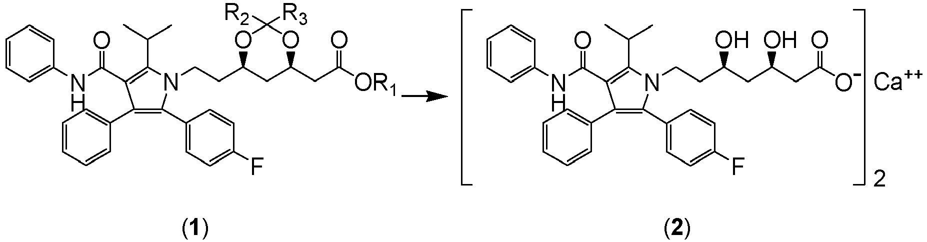

The preparation of atorvastatin is well known and the most common approach is based upon protection/deprotection of the carboxylic acid function as a *tert*-butyl ester ((1); R₁ = -C(CH₃)₃) and of the diol functionality as an acetonide ((1); R₂ = R₃ = -CH₃). This has been described in WO 2008/129562 and in the many references cited therein.

Recently, another type of protection has been disclosed in WO 2009/019561, namely the use of the iso-propyl group for carboxylic acid protection ((1); R₁ = -CH(CH₃)₂). The differences in chemical reactivity of this group as compared to the *tert*-butyl ester function under the prior art deprotection conditions leads to differences in impurity profiles that gave rise to different solutions. In order to further optimize the preparation of atorvastatin there remains room for still different and improved strategies for carboxylic acid protection in atorvastatin synthesis.

Further reference is made to IN 193 427, WO2009/093776, WO2008/075165, Wo2004/096788 and WO89/07598.

### Detailed description of the invention

In a first aspect of the invention, there is provided a compound of the general formula (3) 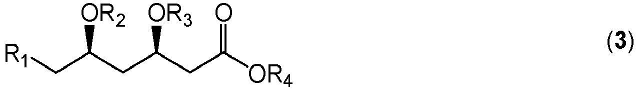 wherein R₁ is -CN or -CH₂NH₂ or a radical of formula (4) or (5) 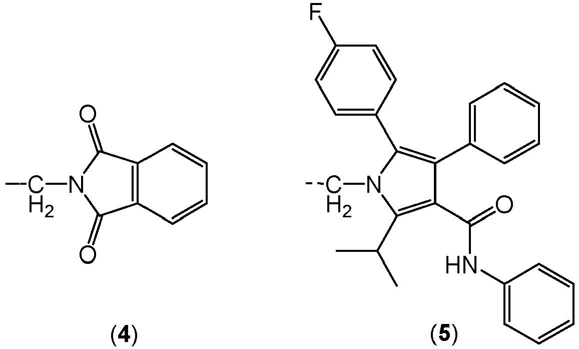 and wherein R₂ and R₃ are hydrogen or combined into a diol protecting group such that the compound of general formula (3) is represented as a compound of general formula **(3a), (3b)** or **(3c)**

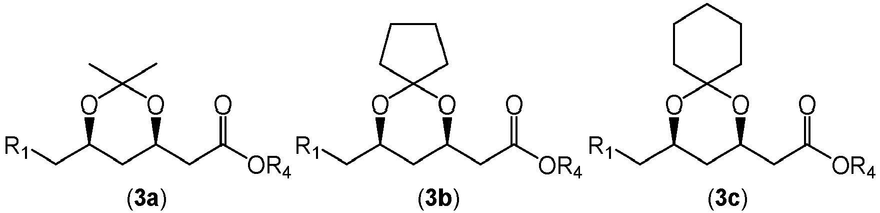

The carboxylic acid protecting group R₄ may be a butyl group such as -CH(CH₃)CH₂CH₃, or a pentyl group such as -CH(CH₃)CH(CH₃)₂ or -CH(CH₃)CH₂CH(CH₃)₂. Combinations may be the compound of general formula (3a) wherein R₁ is -CN and R₄ is -CH(CH₃)CH₂CH₃ or the compound of general formula **(3a)** wherein R₁ is -CH₂NH₂ and R₄ is -CH(CH₃)CH₂CH₃ or the compound of general formula **(3a)** wherein R₁ is a radical of formula **(5)** and R₄ is -CH(CH₃)CH₂CH₃ or the compound of general formula **(3b)** wherein R₁ is -CN and R₄ is -CH(CH₃)CH₂CH₃ or the compound of general formula **(3b)** wherein R₁ is -CH₂NH₂ and R₄ is -CH(CH₃)CH₂CH₃ or the compound of general formula **(3b)** wherein R₁ is a radical of formula **(5)** and R₄ is -CH(CH₃)CH₂CH₃.

It has been found that the compounds of the present invention have advantageous properties during preparative procedures. The respective starting materials required for preparing the esters of the present invention from carboxylic acids, such as alcohols (examples of which are 2-butanol, 3-methyl-2-butanol, 4-methyl-2-pentanol and the like), display a reduced tendency to ignite, burn or explode in comparison with the alcohols used and/or liberated in the prior art (*tert*-butanol and *iso*propanol). This effect has significant positive consequences in view of safety issues related to large scale manufacturing.

Additionally, the alcohols used and/or liberated in the prior art described above (*tert*-butanol and *iso*propanol) are relatively poorly suited for azeotropic removal of water (the azeotrope of these solvents contains approx. 12% water). Removal of water is an important issue in ester synthesis and the alcohols of the present invention are advantageous in that they are significantly better suited for azeotropic water removal. For example the azeotropes of 2-butanol, 3-methyl-2-butanol and 4-methyl-2-pentanol contain 27%, 33% and 43% of water, respectively. Hence, the compounds of the present invention are better suited for application in atorvastatin synthesis when compared to the prior art esters as introduction of the former is more efficient due to the easy of removal of liberated water.

Also, the compounds of the formula **(3a), (3b)** and **(3c),** all with R₁ is -CH₂NH₂, form stable salts with organic acids which is surprising as it is known from prior art that ketals are instable in the presence of acids. It is particularly remarkable that these salts are not only stable at room temperature but remain stable even during recrystallization from an organic solvent carried out at higher temperatures. Accordingly the following acids may be used for salt formation. Aliphatic monocarboxylic acids, dicarboxylic acids or polycarboxylic acids, cycloalkane carboxylic acids, aliphatic unsaturated carboxylic acids, aromatic carboxylic acids, heterocyclic carboxylic acids and sulfonic acids. Examples are acetic acid, butyric acid, valeric acid, isovaleric acid, pivalic acid, oxalic acid, malic acid, succinic acid, malonic acid, citric acid, cyclopropane carboxylic acid, cyclobutane carboxylic acid, cyclopentane carboxylic acid, cyclohexane carboxylic acid, fumaric acid, maleic acid, benzoic acid, *m*-methylbenzoic acid, 4-methoxy-benzoic acid, 4-bromobenzoic acid, 4-*tert*-butylbenzoic acid, benzenesulfonic acid, methanesulfonic acid, *p*-methylbenzenesulfonic acid, *p*-bromobenzenesulfonic acid, nicotic acid, tetrahydrofurane-2-carboxylic acid and thiophen-3-carboxylic acid. Further examples are the oxalic acid salt of compound **(3a,** R₁ is -CH₂NH₂), the pivalic acid salt of compound **(3a,** R₁ is -CH₂NH₂), the oxalic acid salt of compound **(3b,** R₁ is -CH₂NH₂), the pivalic acid salt of compound **(3b,** R₁ is -CH₂NH₂), the oxalic acid salt of compound (3c, R₁ is -CH₂NH₂) and the pivalic acid salt of compound (3c, R₁ is-CH₂NH₂).

In a second aspect the compounds of general formula (3) with R₁ is -CN and R₂, R₃ and R₄ are as described above are prepared by contacting (4*R*,6*S*)-4-hydroxy-6-chloromethyl-tetrahydropyran-2-one with cyanide followed by contacting with an alcohol of formula R₄-OH wherein R₄ is a butyl group such as -CH(CH₃)CH₂CH₃ or a pentyl group such as -CH(CH₃)CH(CH₃)₂ or -CH(CH₃)CH₂CH(CH₃)₂. The resulting product is then reacted to protect the di-hydroxy moiety according to procedures known to the skilled person, such as reaction with acetone or dimethoxypropane and the like.

In a third aspect the compounds of the present invention may be used for the preparation of atorvastatin. One of the stages in atorvastatin synthesis is a Paal-Knorr condensation to form an acetonide ester such as described in, for example, US 5,280,126, US 6,476,235 and US 6,545,153. In WO 2009/023260 it was disclosed that, depending on the conditions, 0.16-0.93% of an amide (dimer) impurity is formed at the Paal-Knorr stage using *tert*-butyl ester protection. The requirements for EU and USP Pharmacopoeia are 0.10% in the final product. Surprisingly it was established that by using the compounds of the present invention, the amount of amide (dimer) impurity is less than 0.10%. This is the more surprising as the commonly used *tert*-butyl ester is generally regarded to be the most stable ester preventing dimerization.

### EXAMPLES

### Example 1

### Esters of (4R,6R)-6-(2-cyanomethyl)-2,2-dimethyl-1,3-dioxane-4-acetic acid

### Preparation of the 1-methylpropyl ester

A 30% solution of NaCN in water (190 g, 1.16 mol) was charged into a reactor. Subsequently, NaOH (1 g, 0.025 mol) and water (133 g) were added and the mixture was stirred for 30 min at 25°C. (4*R*,6*S*)-4-Hydroxy-6-chloromethyl-tetrahydropyran-2-one was added (83 g, 0.46 mol, for preparation see WO2002/06266) in 1 h at 25-30°C. After completion of the addition, stirring was continued for 20 h at 30°C. The reaction mixture was cooled to 20°C and 37% aqueous HCl (85 g) was added in 1 h keeping the temperature < 30°C (the HCN generated at this step was scrubbed into a solution of aqueous sodium hypochlorite). When addition of HCl was completed, stirring was continued for 30 min. The reaction mixture was added to 2-butanol (500 g), stirred for 30 min and heated to 70°C. Under vacuum (∼400 mbar), 500 g of distillate was collected. Then, fresh 2-butanol (500 g) was added and again 500 g of distillate collected. After cooling to 60-65°C, 2-butanol (200 g) was added followed by 37% aqueous HCl (6 g). The reaction mixture was kept stirring for 6 h at 60-65°C. 37% aqueous HCl (2 g) was added, heated to 70°C and under vacuum (400 mbar), 140 g of distillate collected. The reaction mixture was cooled to 25°C and dimethoxypropane (419 g, 4.0 mol) was added. After stirring for 1 h, a saturated aqueous solution of NaHCO₃ (118 g) was added until pH = 6.3. The salts were filtered over dicalite and washed with 2-butanol (2 x 100 g). The obtained solution was concentrated under vacuum. To the resulting brown oil methyl-*tert*-butyl ether (500 g) and water (400 g) were added. The methyl-*tert*-butyl ether was separated and washed with water (3 x 75 g). The methyl-*tert*-butyl phase containing the product was concentrated under vacuum to give the title compound as brown oil (109.7 g, GC assay 81%, yield 71%). This oil was used in the next step (Example 2) without further purification.

### 1,2-Dimethylpropyl ester

For the preparation of the 1,2-dimethylpropyl ester of (4R,6R)-6-(2-cyanomethyl)-2,2-dimethyl-1,3-dioxane-4-acetic acid, it is suggested to repeat the description for the 1-methylpropyl ester, however with replacement of 2-butanol with 3-methyl-2-butanol. Hence, firstly 500 g 2-butanol should be replaced with 600 g 3-methyl-2-butanol (twice) and 200 g 2-butanol should be replaced with 240 g 3-methyl-2-butanol. The dicalite washing step should be carried out with 3-methyl-2-butanol rather than 2-butanol.

### 1,3-Dimethylbutyl ester

For the preparation of the 1,3-dimethylbutyl ester of (4R,6R)-6-(2-cyanomethyl)-2,2-dimethyl-1,3-dioxane-4-acetic acid, it is suggested to repeat the description for the 1-methylpropyl ester, however with replacement of 2-butanol with 4-methyl-2-pentanol. Hence, firstly 500 g 2-butanol should be replaced with 700 g 4-methyl-2-pentanol (twice) and 200 g 2-butanol should be replaced with 275 g 4-methyl-2-pentanol. The dicalite washing step should be carried out with 4-methyl-2-pentanol rather than 2-butanol.

### Example 2

### Preparation of (4R,6R)-6-(2-aminomethyl)-2,2-dimethyl-1,3-dioxane-4-acetic acid 1-methylpropyl ester

(4*R*,6*R*)-6-(2-Cyanomethyl)-2,2-dimethyl-1,3-dioxane-4-acetic acid 1-methylpropyl-ester (54.3 g, assay 81%) was dissolved in 2-butanol (240 g). Raney Cobalt (46.5 g, washed with 2-butanol, 2 x 100 g) was added. The reaction mixture was heated until 50°C and flushed 3 times with nitrogen and with hydrogen. The reaction mass was stirred under 12 bar hydrogen at 50°C or 77 h, then cooled and hydrogenated for another 62 h at 20-25°C. Raney Cobalt was removed by filtration and washed with 2-propanol (3 x 150 g). Concentration under vacuum gave the product as oil (57.4 g, assay 68%, yield 88%). The amine was used in the next step without further purification

### Example 3

### Preparation of 2-((4R,6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid 1-methylpropyl ester

(4*R*,6*R*)-6-(2-Aminomethyl)-2,2-dimethyl-1,3-dioxane-4-acetic acid 1-methylpropyl-ester (50.6 g, assay 68%, 126 mmol) and 2-[2-(4-fluorophenyl)-2-oxo-1-phenylethyl]-4-methyl-3-oxopentanoic acid phenyl amide, (DKT, 57.3 g, 137 mmol) were added to a stirred mixture of heptane (300 g) and tetrahydrofuran (195 g). Pivalic acid (9.1 g, 89 mmol) was added to the slurry and the mixture was heated to reflux under azeotropic water removal. About 150 g of a heptane/tetrahydrofuran/water mixture was distilled in 24 h, which was replaced by addition of fresh heptane (150 g). Azeotropic distillation was continued for 48 h. The temperature increased from initially 78°C to 92°C at the end of the distillation. After cooling to 20-25°C, methyl-*tert*-butylether (300 g) and methanol (16 g) were added. The organic phase containing the product was washed with 0.5 N aqueous NaOH (270 mL) followed by 1N aqueous HCl (270 mL). The organic phase was concentrated under vacuum. The residue was taken up in methanol (430 g) and heated to 40-45°C to give a clear solution. Water (110 g) was added in 2 h, while allowing cooling to 20-25°C. The resulting slurry was stirred for 16 h at 20-25°C. The product was filtered, washed with methanol/water (80/20 v/v, 3 x 30 g). After drying the product was obtained as a white solid (52.3 g, 63% yield).

### Example 4

### Preparation of atorvastatin calcium from 2-((4R,6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid 1-methylpropyl ester

2-((4*R*,6*R*)-6-(2-(3-(Phenylcarbamoyl)-5-(4-fluorophenyl)-2-isopropyl-4-phenyl-1*H-*pyrrol-1-yl)ethyl)-2,2-dimethyl-1,3-dioxan-4-yl)acetic acid 1-methylpropyl ester (6.0 g, 9.1 mmol) was added to methanol (100 g). The mixture was stirred at 37°C until a clear solution was obtained followed by cooling to 30°C. Next, 1 N aqueous HCl (15 mL) was added in 10 min and the resulting reaction mixture stirred for 3h at 25°C. To the mixture, 2.5 N aqueous NaOH (11.3 mL) was added in 15 min at 25-30°C. The mixture was heated to 38°C and stirred for 1h. After cooling to 25°C, the clear solution was concentrated to give 55 g of oily residue. Water (54 g) and methanol (9 g) were added. The aqueous phase was extracted with methyl-*tert*-butyl ether (1 x 28 g and 1 x 18 g) followed by extraction with a mixture of ethylacetate/cyclohexane (21 g ethyl acetate and 18.5 g cyclohexane, 50/50 v/v)). Thereafter, the aqueous phase was treated with 0.6 g active carbon for 15 min. The carbon was removed over a 0.45 µm filter and washed with methanol/water (10 g, 50/50 v/v). The reaction mixture was heated until 48°C and atorvastatin calcium polymorph I seed (0.3 g) added. Then, a solution of Ca-acetate.H₂O (0.9 g) in water (29 g) was added over a period of 90 min. The resulting slurry was heated to 58°C, maintained at this temperature for 30 min and water added (7.5 g). After 1 h, the slurry was cooled to 35°C, kept at this temperature for 2 h and the product isolated by filtration. The resulting wet-cake was slurried in water (75 g) at 40°C. After stirring for 2 h, the product was isolated by filtration and dried at 50°C under vacuum. A white solid was obtained: 4.0 g, Yield 73%, 99.2% pure, Kf water content 4.1%.

## Claims

1. Compound of the general formula (3) 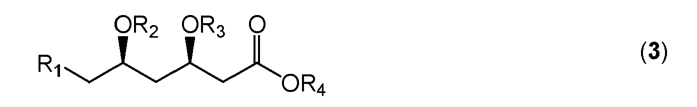 wherein R₁ is -CN or -CH₂NH₂ or a radical of formula (4) or (5) 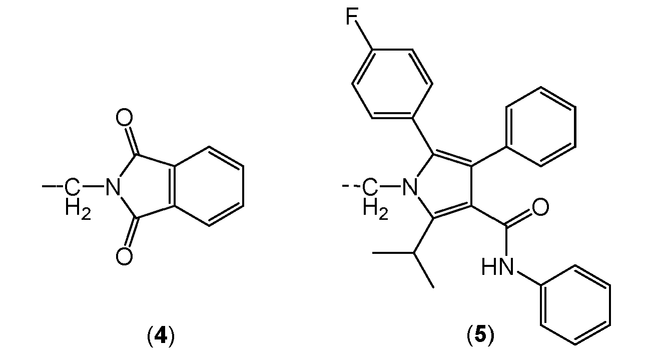 and wherein R₂ and R₃ are hydrogen or combined into a diol protecting group such that the compound of general formula (3) is represented as a compound of general formula (3a), (3b) or (3c) 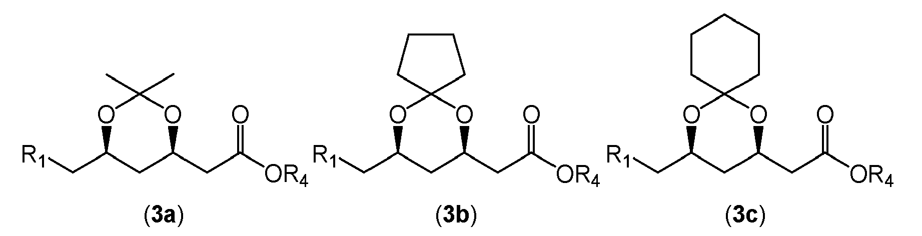 **characterized in that** R₄ is -CH(CH₃)CH₂CH₃, -CH(CH₃)CH(CH₃)₂ or -CH(CH₃)CH₂CH(CH₃)₂.

2. Compound according to claim 1 which is a compound of general formula (3a) wherein R₁ is -CN or -CH₂NH₂ or a radical of formula (5) and R₄ is -CH(CH₃)CH₂CH₃.

3. Compound according to claim 1 wherein R₁ is -CH₂NH₂ which is a salt.

4. Method for the production of a compound of general formula (3) 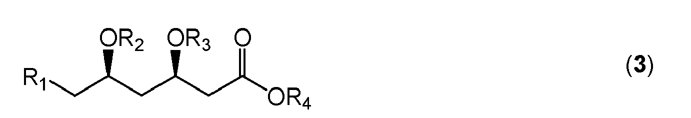 wherein R₁ is -CN or -CH₂NH₂ or a radical of formula **(4)** or **(5)** 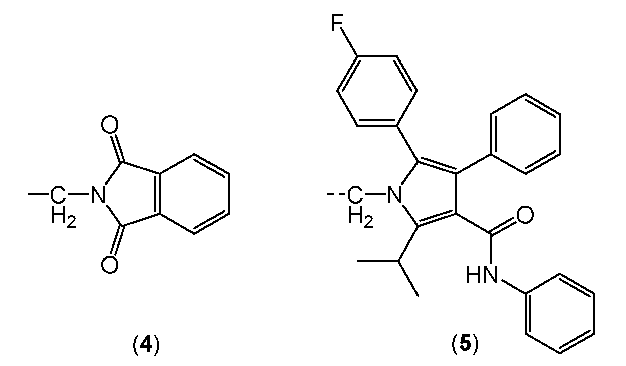 and wherein R₂ and R₃ are hydrogen or combined into a diol protecting group such that the compound of general formula (3) is represented as a compound of general formula (3a), (3b) or (,3c) 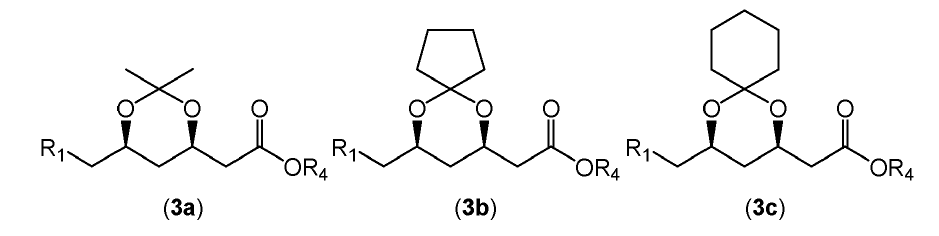 wherein R₄ is -CH(CH₃)CH₂CH₃, -CH(CH₃)CH(CH₃)₂ or -CH(CH₃)CH₂CH(CH₃)₂, comprising contacting a compound of general formula (3) wherein R₁, R₂ and R₃ are as described above and R₄ is hydrogen with an alcohol chosen from the list consisting of 2-butanol, 3-methyl-2-butanol and 4-methyl-2-pentanol.

5. Method according to claim 4 further comprising azeotropic removal of water.

6. Use of a compound of the general formula (3) 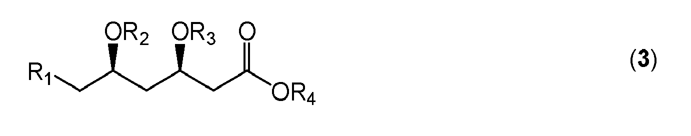 wherein R₁ is -CN or -CH₂NH₂ or a radical of formula **(4)** or **(5)** 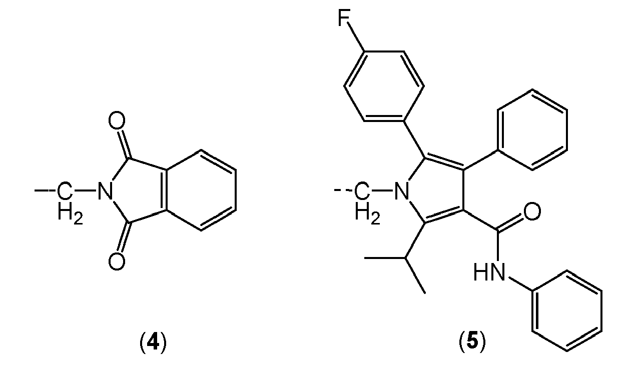 and wherein R₂ and R₃ are hydrogen or combined into a diol protecting group such that the compound of general formula (3) is represented as a compound of general formula **(3a), (3b)** or **(3c)** 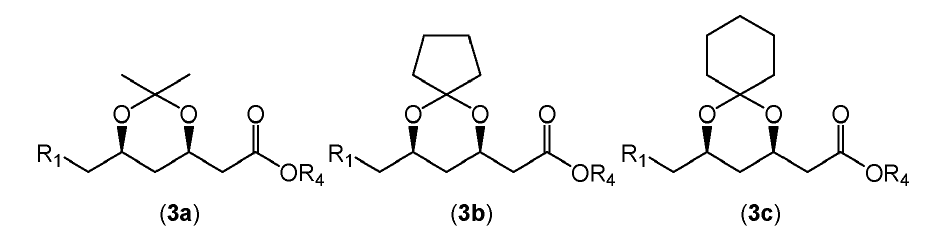 and wherein R₄ is -CH(CH₃)CH₂CH₃, -CH(CH₃)CH(CH₃)₂, or -CH(CH₃)CH₂CH(CH₃)₂ in the preparation of atorvastatin.

## Patentansprüche

1. Verbindung der allgemeinen Formel (3) 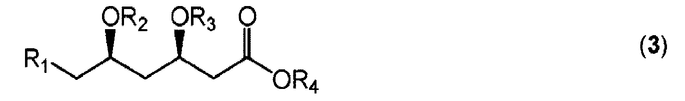 worin R₁ für -CN oder -CH₂NH₂ oder einen Rest der Formel **(4)** oder **(5)** 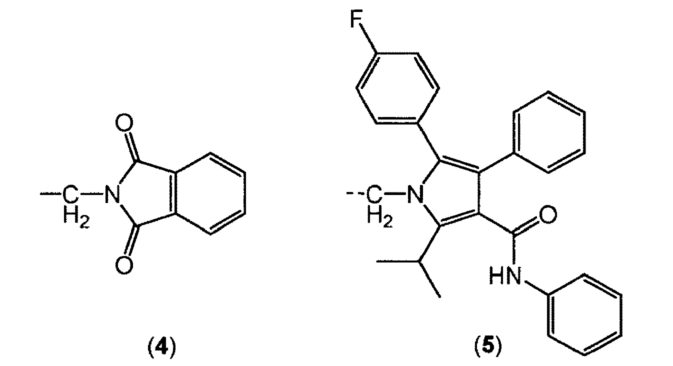 steht und worin R₂ und R₃ für Wasserstoff stehen oder zu einer Diol-Schutzgruppe kombiniert sind, so dass die Verbindung der allgemeinen Formel **(3)** als eine Verbindung der allgemeinen Formel **(3a), (3b)** oder **(3c)** 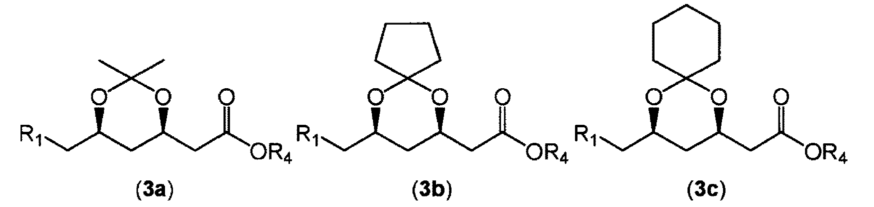 wiedergegeben wird, **dadurch gekennzeichnet, dass** R₄ für -CH(CH₃)CH₂CH₃, -CH(CH₃)CH(CH₃)₂ oder -CH(CH₃)CH₂CH(-CH₃)₂ steht.

2. Verbindung nach Anspruch 1, bei der es sich um eine Verbindung der allgemeinen Formel **(3a)** handelt, worin R₁ für -CN oder -CH₂NH₂ oder einen Rest der Formel **(5)** steht und R₄ für -CH(CH₃)CH₂CH₃ steht.

3. Verbindung nach Anspruch 1, worin R₁ für -CH₂NH₂ steht, bei der es sich um ein Salz handelt.

4. Verfahren zur Herstellung einer Verbindung der allgemeinen Formel **(3)** 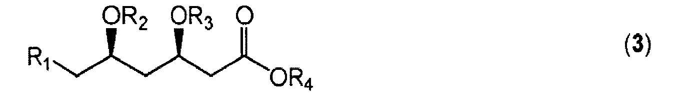 worin R₁ für -CN oder -CH₂NH₂ oder einen Rest der Formel **(4)** oder **(5)** 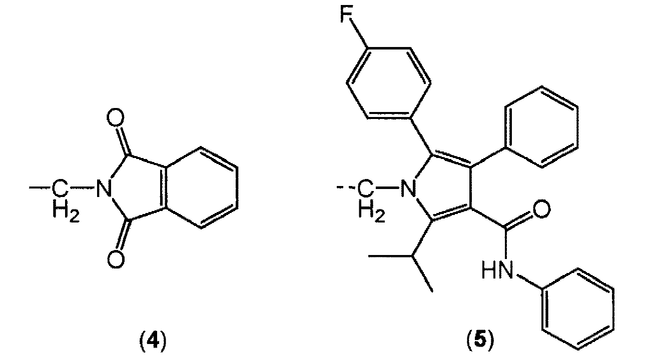 steht und worin R₂ und R₃ für Wasserstoff stehen oder zu einer Diol-Schutzgruppe kombiniert sind, so dass die Verbindung der allgemeinen Formel **(3)** als eine Verbindung der allgemeinen Formel **(3a), (3b)** oder **(3c)** 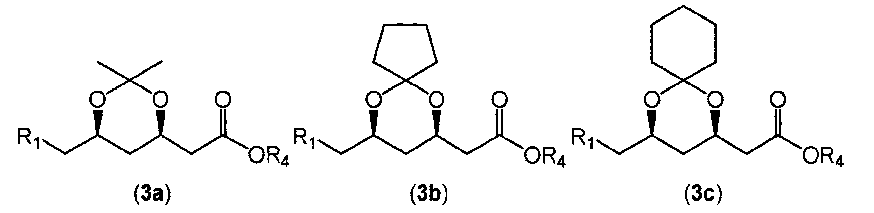 wiedergegeben wird, worin R₄ für -CH(CH₃)CH₂CH₃, -CH(CH₃)CH(CH₃)₂ oder -CH(CH₃)CH₂CH(CH₃)₂ steht, bei dem man eine Verbindung der allgemeinen Formel (**3**), worin R₁, R₂ und R₃ wie oben beschrieben sind und R₄ für Wasserstoff steht, mit einem Alkohol aus der Liste bestehend aus 2-Butanol, 3-Methyl-2-butanol und 4-Methyl-2-pentanol in Berührung bringt.

5. Verfahren nach Anspruch 4, bei dem man ferner Wasser azeotrop entfernt.

6. Verwendung einer Verbindung der allgemeinen Formel (3) 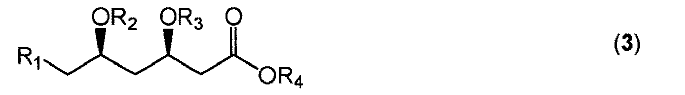 worin R₁ für -CN oder -CH₂NH₂ oder einen Rest der Formel **(4)** oder **(5)** 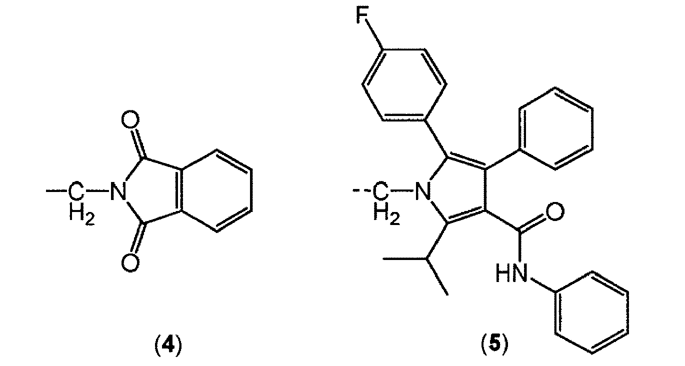 steht und worin R₂ und R₃ für Wasserstoff stehen oder zu einer Diol-Schutzgruppe kombiniert sind, so dass die Verbindung der allgemeinen Formel **(3)** als eine Verbindung der allgemeinen Formel **(3a), (3b)** oder **(3c)** 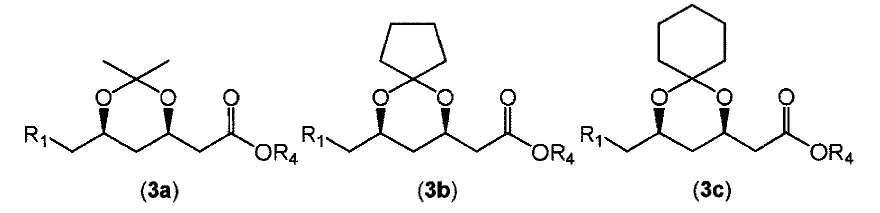 wiedergegeben wird, und worin R₄ für -CH(CH₃)CH₂CH₃, -CH(CH₃)CH(CH₃)₂ oder -CH(CH₃)CH₂CH(CH₃)₂ steht, bei der Herstellung von Atorvastatin.

## Revendications

1. Composé de la formule générale (3) 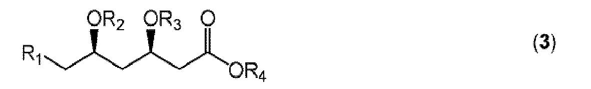 dans laquelle R₁ représente -CN ou -CH₂NH₂ ou un radical de formule **(4)** ou **(5)** 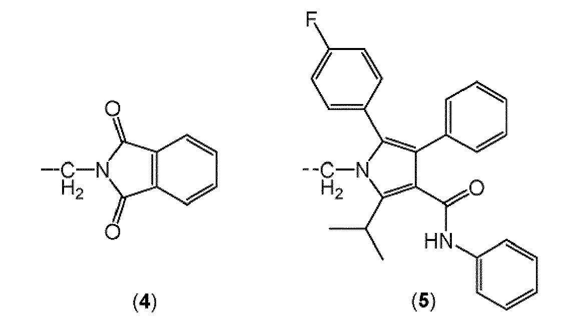 et dans laquelle R₂ et R₃ représentent l'hydrogène ou sont combinés en un groupe protecteur de diol de telle sorte que le composé de formule générale **(3)** est représenté comme un composé de formule générale **(3a), (3b)** ou **(3c)** 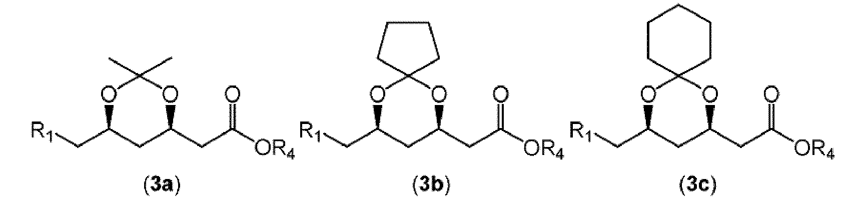 **caractérisé en ce que** R₄ représente -CH(CH₃)CH₂CH₃, -CH(CH₃)CH(CH₃)₂ ou -CH(CH₃)CH₂CH(CH₃)₂.

2. Composé selon la revendication 1 qui est un composé de formule générale **(3a)** dans laquelle R₁ représente -CN ou -CH₂NH₂ ou un radical de formule **(5)** et R₄ représente -CH(CH₃)CH₂CH₃.

3. Composé selon la revendication 1 dans lequel R₁ représente -CH₂NH₂ qui est un sel.

4. Procédé de production d'un composé de formule générale **(3)** 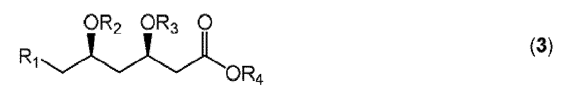 dans laquelle R₁ représente -CN ou -CH₂NH₂ ou un radical de formule **(4)** ou **(5)** 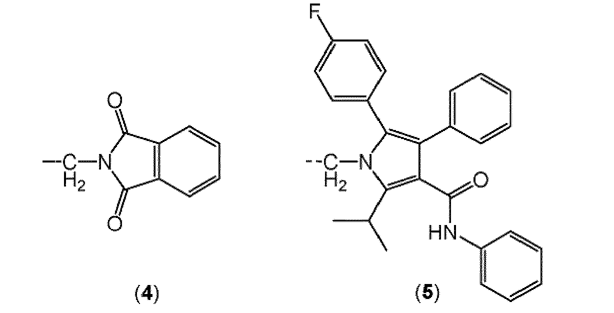 et dans laquelle R₂ et R₃ représentent l'hydrogène ou sont combinés en un groupe protecteur de diol de telle sorte que le composé de formule générale **(3)** est représenté comme un composé de formule générale **(3a), (3b)** ou **(3c)** 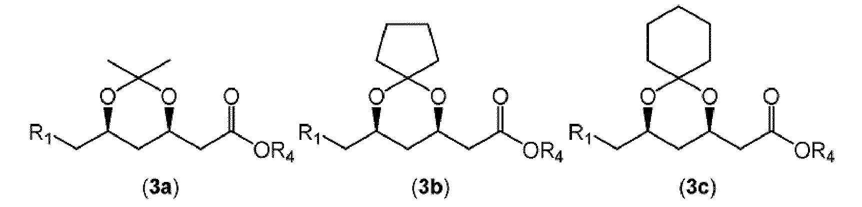 dans lesquelles R₄ représente -CH(CH₃)CH₂CH₃, -CH(CH₃)CH(CH₃)₂ ou -CH(CH₃)CH₂CH(CH₃)₂,
comprenant la mise en contact d'un composé de formule générale **(3)** dans laquelle R₁, R₂ et R₃ sont tels que décrits ci-dessus et R₄ représente l'hydrogène avec un alcool choisi dans la liste constituée par le 2-butanol, le 3-méthyl-2-butanol et le 4-méthyl-2-pentanol.

5. Procédé selon la revendication 4 comprenant en outre le retrait azéotropique de l'eau.

6. Utilisation d'un composé de la formule générale (3) 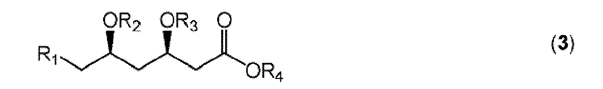 dans laquelle R₁ représente -CN ou -CH₂NH₂ ou un radical de formule **(4)** ou **(5)** 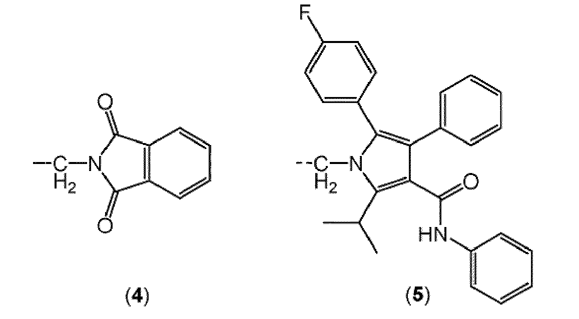 et dans laquelle R₂ et R₃ représentent l'hydrogène ou sont combinés en un groupe protecteur de diol de telle sorte que le composé de formule générale **(3)** est représenté comme un composé de formule générale **(3a), (3b)** ou **(3c)** 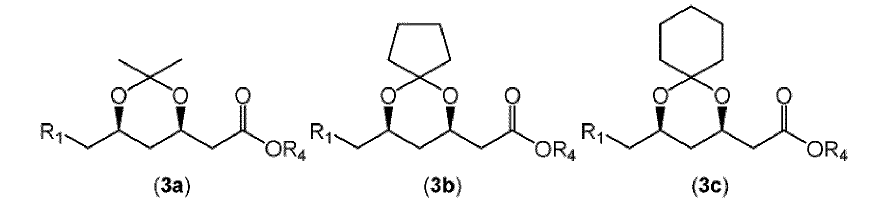 et dans laquelle R₄ représente -CH(CH₃)CH₂CH₃, -CH(CH₃)CH(CH₃)₂ ou -CH(CH₃)CH₂CH(CH₃)₂ dans la préparation d'atorvastatine.
